# EUROPEAN PATENT SPECIFICATION

(11) **EP 2 380 552 B1**
(45) Date of publication and mention of the grant of the patent: **10.10.2018**
(21) Application number: 10733396.5
(22) Date of filing: 05.01.2010
(51) Int. Cl.: A61K 8/06, A61K 8/31, A61K 8/39, A61K 8/60, A61Q 1/00, A61Q 1/04, A61Q 17/04, A61Q 19/00, B01J 13/00, A23L 29/10, A23L 5/42, A23L 33/105

(54) **EMULSION COMPOSITION, AND USES OF THE EMULSION COMPOSITION**
EMULSIONSZUSAMMENSETZUNG UND VERWENDUNGEN VON DER EMULSIONSZUSAMMENSETZUNG
COMPOSITION D'ÉMULSION, ET UTILISATIONS DE LADITE COMPOSITION D'ÉMULSION

(30) Priority: 20.01.2009 JP 2009009688
(43) Date of publication of application: 26.10.2011
(73) Proprietor: FUJIFILM Corporation, Tokyo 106-8620 (JP)
(72) Inventor: TANISAKA, Hiroki, Ashigarakami-gun Kanagawa 258-8577 (JP)
(74) Representative: Klunker IP Patentanwälte PartG mbB
(86) International application number: PCT/JP2010/050035
(87) International publication number: WO 2010/084789

(56) References cited:
- EP-A1- 1 609 779
- WO-A1-2007/009601
- JP-A- 10 263 385
- JP-A- 2000 106 844
- JP-A- 2005 075 817
- JP-A- 2007 326 829
- JP-A- 2007 326 829
- JP-A- 2008 063 476
- JP-A- 2008 154 577
- JP-A- 2008 169 117

## Description

### TECHNICAL FIELD

The invention relates to an emulsion composition and uses of the emulsion composition for the preparation of a beverage or a cosmetic product.

### BACKGROUND ART

Oily ingredients have been added to aqueous beverages, aqueous food products, and aqueous cosmetics. However, since oily ingredients are insoluble or hardly soluble in water, oily ingredients have generally been dispersed in aqueous media by means of emulsification or the like so that so-called emulsions are formed. Emulsions scatter light, depending on the particle diameter. Therefore, when the particle diameter is large, emulsions and food or cosmetic products produced by the addition of the emulsions have turbidity, which may be undesirable in terms of appearance. Therefore, making the emulsion particle diameter very small to such a level that light scattering is very low has been desired.

On the other hand, carotenoids have been added to food products, cosmetics, pharmaceuticals, etc. Such carotenoids, which are widely known as hardly-soluble materials, are generally emulsified before use.

Specific examples of known carotenoid-containing emulsion compositions include a carotenoid-containing emulsion composition that contains at least one water-soluble emulsifier in the aqueous phase and tocopherol and lecithin in the oil phase (see Japanese Patent Application Laid-Open (JP-A) No. 2008-13751); a solubilized carotenoid colorant-containing liquid preparation for food that includes fine particles of a composition of a food carotenoid colorant and a polyglycerol fatty acid ester and has a transmittance of 99% or more at 660 nm when the absorbance is 1 at the absorption maximum wavelength in the visible region (see JP-A No. 10-120933); and a carotenoid-containing composition that is produced by emulsifying an oil phase of a solution of a carotenoid in an oil or fat into an aqueous phase containing a polyhydric alcohol in the presence of a polyglycerol fatty acid ester and lecithin, and has an average oil-phase particle diameter of 100 nm or less (see JP-A No. 09-157159).

JP-A- No. 2008-169117 describes a dispersion composition containing a carotenoid component, a phospholipid and ascorbic acid and having good storage stability and a cosmetic for skin care using the same.

Carotenoids also include those having a melting point of 100°C or more as measured in the presence of an oil (hereinafter referred to as "high-melting-point carotenoid" as appropriate).

When such carotenoids are used, using the technique described in the above documents, it has been difficult to obtain a highly transparent emulsion composition that contains dispersed particles with a small particle diameter and can keep the small particle diameter stable for a long term.

### SUMMARY OF INVENTION

### SOLUTION TO PROBLEM

An object of the invention is to provide a high-melting-point-carotenoid-containing emulsion composition that contains dispersed particles with a small particle diameter, has high storage stability, and is highly transparent.

Another object of the invention is the use of the emulsion composition to provide a beverage or a cosmetic product, the emulsion composition being a high-melting-point-carotenoid-containing emulsion composition that contains dispersed particles with a small particle diameter, has high storage stability, and is highly transparent.

A first aspect of the invention provides an emulsion composition obtained by mixing an oil phase containing a carotenoid having a melting point of 100 °C or more as detected when it is contained in an oil; and an aqueous phase containing a polyglycerol fatty acid ester and a nonionic emulsifier that is different from the polyglycerol fatty acid ester, wherein a weight ratio (A)/(B) of an amount (A) of polyglycerol fatty acid ester contained in the emulsion composition to an amount (B) of nonionic emulsifier other than polyglycerol fatty acid ester, that is contained in the emulsion composition is in a range of 2.5 to 10, and a particle diameter of dispersed particles that contain the carotenoid is 200 nm or less.

A second aspect of the invention provides the use of the above-captioned emulsion composition for the production of a beverage.

A third aspect of the invention provides the use of the above-captioned emulsion composition for the production of a cosmetic product selected from the group consisting of: a skin lotion, an essence, a milky lotion, a cream pack, a mask, a pack, a shampoo cosmetic, a fragrance cosmetic, a liquid body cleanser, a UV-care cosmetic, a deodorant cosmetic and an oral care cosmetic.

According to the invention, it is possible to provide a high-melting-point-carotenoid-containing emulsion composition that contains dispersed particles with a small particle diameter, has high storage stability, and is highly transparent.

According to the invention, it is also possible to provide food and cosmetic products selected from the group consisting of: a skin lotion, an essence, a milky lotion, a cream pack, a mask, a pack, a shampoo cosmetic, a fragrance cosmetic, a liquid body cleanser, a UV-care cosmetic, a deodorant cosmetic and an oral care cosmetic using the high-melting-point-carotenoid-containing emulsion composition that contains dispersed particles with a small particle diameter, has high storage stability, and is highly transparent.

### DESCRIPTION OF EMBODIMENTS

### <Emulsion Composition>

The emulsion composition of the invention is obtained by mixing an oil phase containing a carotenoid having a melting point of 100°C or more as detected when it is contained in an oil, and an aqueous phase containing a polyglycerol fatty acid ester and a nonionic emulsifier different from the polyglycerol fatty acid ester. A weight ratio (A)/(B) of an amount (A) of the polyglycerol fatty acid ester to an amount (B) of the nonionic emulsifier different from the polyglycerol fatty acid ester is in the range of 2.5 to 10, and a particle diameter of dispersed particles that contain the carotenoid is 200 nm or less.

As stated above, using the oil phase containing a high-melting-point carotenoid and the aqueous phase in which two or more emulsifiers including a polyglycerol fatty acid ester and a nonionic emulsifier different from the polyglycerol fatty acid ester are used in a specific mass ratio, it is possible to make the particle diameter of dispersed particles containing the carotenoid small, keep the resulting emulsion composition in such a state in which dispersed particles have a small particle diameter for a long time, and provide an emulsion composition having high transparency.

In the description, the term "step" is intended to include not only an independent step but also a step not clearly distinguishable from other steps as long as the intended effect of the step is achieved.

In the description, the value range expressed with "from ....to ..." refers to a range including the values preceding and following "to" as minimum and maximum values.

In the description, unless otherwise specified, the content or amount of each ingredient in the composition means the content or amount of a single material when in the composition, there is only one material corresponding to each ingredient defined in the description, or means the total content or amount of two or more materials when in the composition, there are two or more materials corresponding to each ingredient defined in the description.

The emulsion composition of the invention is an oil-in-water emulsion including carotenoid-containing particles dispersed as the oil phase in the aqueous phase. In the composition, oil-phase ingredients that form the oil phase may be in the form of completely dissolved oil droplets or in the form of partially insoluble solids. In the description, such oil droplets and solids are generically called dispersed particles.

Hereinafter, each ingredient of the emulsion composition of the invention will be described.

### [Oil Phase-Forming Ingredients]

### [High-Melting-Point Carotenoid]

The emulsion composition according to the invention contains, in the oil phase (dispersed particles), a carotenoid having a melting point of 100°C or more as measured when it is contained in an oil (high-melting-point carotenoid).

The high-melting-point carotenoid refers to a carotenoid having a melting point of 100°C or more, which is measured and detected through the following steps (1) to (4).
(1) A carotenoid-containing oil-based sample containing an oil and from 1 to 50% by mass of a carotenoid is prepared.
(2) The prepared oil-based sample is heated using METTLER FP900 Thermal Analysis System and FP82 Hot Stage (manufactured by Mettler-Toledo International Inc.). The heating is performed under the conditions of an initial temperature of 30°C and a rate of temperature rise of 5°C/minute.
(3) In the heat treatment, the melting behavior of the carotenoid crystal in the sample is observed with an optical microscope ECLIPSE LV100POL (manufactured by Nikon Corporation). The observation is performed at a magnification of 200 times in the dark field using a polarizing filter.
(4) The temperature at which the crystal appearing bright in the dark field is completely melted is recorded as the melting point.

The composition of the oil-based sample used in the measurement method is preferably in accordance with the composition of the oil phase of the emulsion composition to be prepared, and the carotenoid may be diluted with any other oil material as long as the carotenoid concentration exceeds 1% by mass. The oil material to be used for dilution is preferably an oil or a fat, but it is not restricted to an oil or a fat and the composition of the oil-based sample only has to contain an oil material that may be added to the emulsion composition described below. When the carotenoid is extracted in the form of a carotenoid-containing oil from a natural product, the carotenoid-containing oil extracted from the natural product may be used as the oil-based sample in the measurement method and directly subjected to the melting point measurement. Any known oil may be directly used without restriction in the extraction from the natural product.

While the melting point of the carotenoid in an oil-based sample may be similarly measured using a commercially available melting point meter or differential scanning calorimeter, in the present invention, a measurement method including the steps (1) to (4) is adopted.

In the present invention, any carotenoid having a melting point of 100°C or more as detected by the above measurement may be used.

Examples of the high-melting-point carotenoid include lycopene, α-carotene, β-carotene, γ-carotene, and non-esterified xanthophylls. Examples of such xanthophylls include astaxanthin, fucoxanthin, lutein, zeaxanthin, capsanthin, β-cryptoxanthin, and violaxanthin. These high-melting-point carotenoids may be used alone or in a mixture of two or more. Esterified xanthophylls, which have increased fat-solubility due to the bonded medium or long acyl chain, are known to have a low crystal melting point. In the present invention, therefore, a non-esterified xanthophyll is used as the high-melting-point carotenoid.

In particular, lycopene is preferred, because it is known to have a high antioxidant effect, a skin-whitening effect, or the like and the addition of it to food products, cosmetics, pharmaceutical raw materials, and processed products thereof has been demanded, examined, and practiced.

Lycopene is a carotenoid of the chemical formula C₄₀H₅₆ (536.87 in molecular weight), which belongs to carotene family, a kind of carotenoids, and is a red coloring matter showing an absorption maximum at 474 nm (acetone).

Lycopene may be present in the form of cis- or trans-isomers with respect to conjugate double bonds at the center of the molecule. Examples include an all-trans form, a 9-cis form, and a 13-cis form.

The emulsion composition of the invention may contain lycopene in the form of a lycopene-containing oil, which is separated or extracted from a natural product containing it.

In nature, tomato, persimmon, watermelon, and pink grapefruit contain lycopene, and the lycopene-containing oil may be separated or extracted from these natural products.

Lycopene for use in the invention may be such an extract, a product produced by appropriate purification of such an extract as needed, or a synthetic product.

In the invention, lycopene extracted from tomato is particularly preferred in view of quality and productivity.

In the invention, a tomato extract, which is widely commercially available, may be used as the lycopene-containing oil. Examples of such a commercially available lycopene-containing oil include Lyc-O-Mato 15% and Lyc-O-Mato 6% available from SUN BRIGHT CO., LTD. and LYCOPENE 18 available from KYOWA HAKKO BIO Co., Ltd.

The total high-melting-point carotenoid content is preferably from 0.1% by mass to 5% by mass, more preferably from 0.2% by mass to 3% by mass, even more preferably from 0.3% by mass to 2% by mass, based on the total weight of the emulsion composition.

### [Additional Oily Ingredients]

In an embodiment of the invention, the oil phase may also contain an additional oily ingredient or ingredients other than the high-melting-point carotenoid. The additional oily ingredient may be of any ingredient which is not soluble in an aqueous medium but soluble in an oil medium.

Hereinafter, a description is given of additional oily ingredients.

In the invention, one of additional oily ingredients may be the above-mentioned oil for use in the detection of the melting point of the high-melting-point carotenoid. When an oil containing the high-melting-point carotenoid is commercially available, such a commercially available product may be directly used as a component of the oil phase according to the invention.

Besides the above oil for use in the detection of the melting point of the high-melting-point carotenoid, preferable examples of the additional oily ingredients include carotenoids other than the high-melting-point carotenoid, phospholipids, unsaturated fatty acids, oils and fats such as coconut oil, antioxidants including oil-soluble vitamins such as tocopherol, and ubiquinones.

### (Carotenoids)

Carotenoids including natural coloring matters, exclusive of the high-melting-point carotenoids, are preferably used as additional oily ingredients.

Carotenoids for use in the emulsion composition of the invention include coloring matters of yellow to red terpenoids, which include those derived from plants, algae, and bacteria.

Carotenoids are not limited to naturally-occurring carotenoids, and in the invention, carotenoids also include those obtained by conventional techniques. For example, many carotenes belonging to the carotenoids described below are produced by synthesis, and many of commercially-available β-carotene products are produced by synthesis.

Carotenoids other than the high-melting-point carotenoid refer to those having a melting point of less than 100°C as measured by the above method, examples of which include hydrocarbons (carotenes) and oxidized alcohol derivatives thereof (xanthophylls).

Examples of carotenoids include actinioerythrol, astaxanthin (esterified form), bixin, canthaxanthin, capsanthin (esterified form), capsorbin, β-8'-apocarotenal (apocarotenal), β-12'-apocarotenal, β-cryptoxanthin (esterified form), lutein (esterified form), violaxanthin (esterified form), zeaxanthin (esterified form), and esters of hydroxyl- or carboxyl-containing compounds selected from the above.

In nature, many carotenoids are present in the form of cis- and trans-isomers, but synthetic carotenoids are often a mixture of cis and trans forms.

Carotenoids can be usually extracted from plant materials. Such carotenoids have various functions. For example, lutein, which is extracted from marigold petals, is widely used as a raw material for poultry feeds and has the function of coloring poultry skin and fat and poultry eggs.

When the high-melting-point carotenoid is used in combination with a carotenoid or carotenoids other than the high-melting-point carotenoid, the content of the high-melting-point carotenoid in all carotenoids is preferably 10% by mass or more, more preferably 30% by mass or more.

### (Unsaturated Fatty Acid)

Examples of unsaturated fatty acids as the additional oily ingredients include monounsaturated fatty acids (ω-9, such as oleic acid) and polyunsaturated fatty acids (ω-3 and ω-6).

Among the polyunsaturated fatty acids, examples of ω-3 oils and fats include linolenic acid (including linseed oil etc.), eicosapentaenoic acid (EPA), docosahexaenoic acid (DHA), and fish oils containing any of them.

DHA is an abbreviation of docosahexaenoic acid, which is a generic name for carboxylic acids having a 22-carbon chain with six double bonds (22:6). It is useful because it usually has cis double bonds at all the 4, 7, 10, 13, 16, and 19 positions, which are biologically important.

### (Oils and Fats)

Among the additional oily ingredients, examples of oils and fats other than ω-3 oils and fats include oils and fats (fatty oils) being liquid at room temperature and oils and fats (fats) being solid at room temperature.

Besides the above oil for use in the detection of the melting point of the high-melting-point carotenoid, examples of the liquid oils and fats include triglycerol and medium-chain fatty acid triglycerides such as glycerol trioctanoate and glycerol triisopalmitate. Examples include olive oil, camellia oil, macadamia nut oil, castor oil, avocado oil, evening primrose oil, turtle oil, corn oil, mink oil, rapeseed oil, egg yolk oil, sesame oil, persic oil, wheat germ oil, sasanqua oil, linseed oil, safflower oil, cotton seed oil, perilla oil, soybean oil, peanut oil, tea seed oil, kaya oil, rice bran oil, china wood oil, tung oil, hohoba oil, germ oil, salad oil, safflower oil, palm oil, coconut oil, peanut oil, almond oil, hazelnut oil, walnut oil, and grape seed oil.

Examples of the solid oils and fats include beef tallow, hydrogenated beef tallow, neat's foot oil, beef bone oil, mink oil, egg yolk oil, lard, horse fat, mutton tallow, hydrogenated oil, cacao oil, coconut oil, hydrogenated coconut oil, palm oil, palm hydrogenated oil, Japan tallow, Japan tallow kernel oil, and hydrogenated castor oil, waxes, and higher alcohols.

Among the above, medium-chain fatty acid triglycerides are preferred in view of particle diameter and stability of the emulsion composition, and specifically, coconut oil is preferably used.

In the invention, commercially available oils and fats may be used.

In the invention, one kind of the oils and fats may be used singly or two or more thereof may be used in a mixture.

### (Antioxidant)

In the invention, a fat-soluble antioxidant having a radical-scavenging function is preferably added as an additional oily ingredient.

In a preferred mode, a single antioxidant may be used singly as an oily ingredient, or an antioxidant may be used in combination with any other oily ingredient to prevent oxidation of the other oily ingredient.

An antioxidant is an additive that inhibits the generation of radicals and traps the generated radicals as quickly as possible to play a role in blocking chain reaction (reference: "Yukagaku Binran (A Handbook of Oil Chemistry), 4th ed.," edited by Japan Oil Chemists' Society, 2001).

Known methods for directly checking whether a material functions as an antioxidant include a method in which the material is mixed with a reagent and how the material traps radicals is measured by means of a spectrophotometer or an ESR (electron spin resonance) spectrometer. In such methods, DPPH (1,1-diphenyl-2-picrylhydrazyl) or galvinoxyl radical is used as the reagent.

In the invention, a compound with which the time required to increase the peroxide value (POV) of an oil or fat to 60 meq/kg through auto-oxidation of the oil or fat under the experimental conditions below is twice or more that required for a blank, is defined as an "antioxidant." The peroxide value (POV) of an oil or fat is measured by a conventional method.

### <Conditions>

Oil or fat: Olive oil
Amount of sample added: 0.1% by mass based on the amount of the oil or fat
Test method: The sample is heated at 190°C, while the POV is measured over time by a conventional method, and the time required for the POV to reach 60 meq/kg is calculated.

In the invention, the antioxidant is preferably such that the time required for the POV to reach 60 meq/kg is five times or more that required for a blank, in view of stability of the emulsion against oxidation.

The antioxidant which can be used in the invention may be any compound capable of functioning as the antioxidant, among the various antioxidants described in "Kosankazai no Riron to Jissai (Theory and Practice of Antioxidants)" written by Kajimoto and published by San Shobo (1984) and Sanka Boshizai Handobukku (Handbook of Antioxidants) written by Sawatari, Nishino, and Tabata and published by Taiseisha (1976). Specific examples include compounds having a phenolic OH group, amine compounds such as phenylenediamine, oil-solublilized derivatives of ascorbic acid and oil-solublilized derivatives of erythorbic acid.

Preferred examples of the antioxidant are shown below, which are not intended to limit the scope of the invention.

Examples of compounds having a phenolic OH group include polyphenols (such as catechin contained in a tea extract), guaiac gum, nordihydroguaiaretic acid (NDGA), gallic esters, BHT (butylhydroxytoluene), BHA (butylhydroxyanisol), carnosic acids (such as a rosemary extract), ferulic acid, vitamin E compounds, and bisphenols. Examples of gallic esters include propyl gallate, butyl gallate, and octyl gallate.

Examples of amine compounds include phenylenediamine, diphenyl-p-phenylenediamine, and 4-amino-p-diphenylamine. Diphenyl-p-phenylenediamine or 4-amino-p-diphenylamine is more preferred.

Examples of oil-solubilized derivatives of ascorbic acid and oil-solubilized derivatives erythorbic acid include L-ascorbyl stearate, L-ascorbyl tetraisopalmitate, L-ascorbyl palmitate, erythorbyl palmitate, and erythorbyl tetraisopalmitate.

In particular, vitamin E compounds are preferably used because they have a high level of safety and antioxidant function.

Examples of vitamin E compounds include, but are not particularly limited to, those selected from a group of compounds consisting of tocopherol and derivatives thereof and a group of compounds consisting of tocotrienol and derivatives thereof. One of these compounds may be used singly or two or more compounds may be used in combination. Compounds selected from the group of compounds consisting of tocopherol and derivatives thereof and the group of compounds consisting of tocotrienol and derivatives thereof, respectively, may also be used in combination.

The group of compounds consisting of tocopherol and derivatives thereof include d1-α-tocopherol, d1-β-tocopherol, d1-γ-tocopherol, d1-δ-tocopherol, d1-α-tocopherol acetate, d1-α-tocopherol nicotinate, d1-α-tocopherol linoleate, and d1-α-tocopherol succinate. Among them, more preferred are d1-α-tocopherol, d1-β-tocopherol, d1-γ-tocopherol, d1-δ-tocopherol, and mixtures thereof (mixed tocopherol). Acetic acid esters thereof are preferably used as tocopherol derivatives.

The group of compounds consisting of tocotrienol and derivatives thereof include α-tocotrienol, β-tocotrienol, γ-tocotrienol, and δ-tocotrienol. Acetic acid esters thereof are preferably used as tocotrienol derivatives. Tocotrienol is a tocopherol analog compound contained in wheat, barley, rice bran, palm oil, etc., and has three double bonds in a tocopherol side chain moiety with and has high antioxidant performance.

These vitamin E compounds are preferably contained, as oil-soluble antioxidants, particularly in the oil phase of the emulsion composition of the invention, so that they can effectively perform the function of preventing oxidation of oily ingredients. Among the vitamin E compounds, at least one selected from a group of compounds consisting of tocotrienol and derivatives thereof is more preferably contained in view of antioxidant effect.

The total antioxidant content of the emulsion composition is generally from 0.001% by mass to 20.0% by mass, preferably from 0.01% by mass to 10.0% by mass, more preferably from 0.1% by mass to 5.0% by mass, based on the amount of the emulsion composition.

### (Ubiquinones)

Ubiquinones may be used as additional oily ingredients in the invention. Examples of ubiquinones include coenzyme Q series such as coenzyme Q10. Coenzyme Q10 is a coenzyme listed as ubidecarenone in Japan Pharmacopoeia, and may also be called ubiquinone 10 or coenzyme UQ10. In nature, coenzyme Q10 is contained in natural sources such as yeast, mackerel, sardine, and wheat germ, in a relatively large amount, and may be extracted with a solvent such as hot water, aqueous alcohol, or acetone. It can be industrially produced, and a fermentation process and a synthetic process are generally known. Coenzyme Q10 for use in the invention may be an extract from a natural product or an industrially synthesized product. Commercially available coenzyme Q10 may be used, examples of which include COENZYME Q10 manufactured by Nisshin Pharma Inc. and COENZYME Q10 POWDER manufactured by NOF CORPORATION.

The total oily ingredient content of the emulsion composition of the invention is preferably from 0.1% by mass to 50% by mass, more preferably from 0.5% by mass to 25% by mass, even more preferably from 0.2% by mass to 10% by mass in view of applications of the emulsion composition and the particle diameter and the storage stability of thereof.

When the total oily ingredient content is 0.1% by mass or more, application of the emulsion composition to food products and cosmetics is tend to be easy while avoiding the content of active ingredients being insufficient, and when the total oily ingredient content is 50% by mass or less, an increase in particle diameter or a reduction in emulsion stability is tend to be less likely to occur.

### [Polyglycerol Fatty Acid Ester]

The emulsion composition of the invention contains a polyglycerol fatty acid ester.

The polyglycerol fatty acid ester in the invention may be an ester of polyglycerol with an average degree of polymerization of 2 or more, preferably from 6 to 15, more preferably from 8 to 10, with a fatty acid of from 8 to 18 carbon atoms, such as caprylic acid, capric acid, lauric acid, myristic acid, palmitic acid, stearic acid, oleic acid, or linoleic acid.

Preferred examples of the polyglycerol fatty acid ester include hexaglyceryl monooleate, hexaglyceryl monostearate, hexaglyceryl monopalmitate, hexaglyceryl monomyristate, hexaglyceryl monolaurate, decaglyceryl monooleate, decaglyceryl monostearate, decaglyceryl monopalmitate, decaglyceryl monomyristate, and decaglyceryl monolaurate.

Among them, more preferred are decaglycerol monoolate (HLB=12), decaglyceryl monostearate (HLB=12), decaglyceryl monopalmitate (HLB=13), decaglycerol monomyristate (HLB=14), and decaglyceryl monolaurate (HLB=16).

These polyglycerol fatty acid esters may be used alone or in any combination.

Herein, HLB is hydrophilicity-hydrophobicity balance usually used in the field of surfactants, and can be calculated using a generally used calculation formula such as Kawakami's formula. In the invention, the Kawakami's formula shown below is used.

HLB = 7 + 11.7log (M_{w}/Mₒ), wherein M_{w} represents the molecular weight of a hydrophilic group, and Mₒ represents the molecular weight of a hydrophobic group.

HLB values shown in catalogs or others may be used.

As seen from the formula, compounds with the arbitrary HLB value can be obtained utilizing the additivity of HLB.

Examples of commercially available polyglycerol fatty acid esters include NIKKOL DGMS, NIKKOL DGMO-CV, NIKKOL DGMO-90V, NIKKOL DGDO, NIKKOL DGMIS, NIKKOL DGTIS, NIKKOL TETRAGLYN 1-SV, NIKKOL TETRAGLYN 1-O, NIKKOL TETRAGLYN 3-S, NIKKOL TETRAGLYN 5-S, NIKKOL TETRAGLYN 5-O, NIKKOL HEXAGLYN 1-L, NIKKOL HEXAGLYN 1-M, NIKKOL HEXAGLYN 1-SV, NIKKOL HEXAGLYN 1-O, NIKKOL HEXAGLYN 3-S, NIKKOL HEXAGLYN 4-B, NIKKOL HEXAGLYN 5-S, NIKKOL HEXAGLYN 5-O, NIKKOL HEXAGLYN PR-15, NIKKOL DECAGLYN 1-L, NIKKOL DECAGLYN 1-M, NIKKOL DECAGLYN 1-SV, NIKKOL DECAGLYN 1-50SV, NIKKOL DECAGLYN 1-ISV, NIKKOL DECAGLYN 1-O, NIKKOL DECAGLYN 1-OV, NIKKOL DECAGLYN 1-LN, NIKKOL DECAGLYN 2-SV, NIKKOL DECAGLYN 2-ISV, NIKKOL DECAGLYN 3-SV, NIKKOL DECAGLYN 3-OV, NIKKOL DECAGLYN 5-SV, NIKKOL DECAGLYN 5-HS, NIKKOL DECAGLYN 5-IS, NIKKOL DECAGLYN 5-OV, NIKKOL DECAGLYN 5-O-R, NIKKOL DECAGLYN 7-S, NIKKOL DECAGLYN 7-O, NIKKOL DECAGLYN 10-SV, NIKKOL DECAGLYN 10-IS, NIKKOL DECAGLYN 10-OV, NIKKOL DECAGLYN 10-MAC, and NIKKOL DECAGLYN PR-20, which are manufactured by Nikko Chemicals Co., Ltd.; RYOTO-POLYGLYESTERs, L-7D, L-10D, M-10D, P-8D, SWA-10D, SWA-15D, SWA-20D, S-24D, S-28D, O-15D, O-50D, B-70D, B-100D, ER-60D, LOP-120DP, DS13W, DS3, HS11, HS9, TS4, TS2, DL15, and DO13, which are manufactured by Mitsubishi-Kagaku Foods Corporation; SUN SOFT Q-17UL, SUN SOFT Q-14S, and SUN SOFT A-141C, which are manufactured by Taiyo Kagaku Co., Ltd.; and POEM DO-100 and POEM J-0021, which are manufactured by RIKEN VITAMIN CO., LTD.:.

Among the above, preferred are NIKKOL DECAGLYN 1-L, NIKKOL DECAGLYN 1-M, NIKKOL DECAGLYN 1-SV, NIKKOL DECAGLYN 1-50SV, NIKKOL DECAGLYN 1-ISV, NIKKOL DECAGLYN 1-O, NIKKOL DECAGLYN 1-OV, NIKKOL DECAGLYN 1-LN, RYOTO-POLYGLYESTERs L-7D, L-10D, M-10D, P-8D, SWA-10D, SWA-15D, SWA-20D, S-24D, S-28D, 0-15D, O-50D, B-70D, B-100D, ER-60D, and LOP-120DP.

If the total polyglycerol fatty acid ester content is too low, particles with a small particle diameter may fail to be obtained, and the emulsion may have insufficient stability, and if it is too high, a certain problem may occur, such as vigorous foaming of the emulsion. Therefore, the total polyglycerol fatty acid ester content of the emulsion composition is preferably from 0.1% by mass to 20% by mass, more preferably from 1% by mass to 18% by mass, even more preferably from 5% by mass to 15% by mass.

### [Nonionic Emulsifier Different From Polyglycerol Fatty Acid Ester]

The emulsion composition of the invention contains a nonionic emulsifier different from the polyglycerol fatty acid ester.

In the invention, the nonionic emulsifier different from the polyglycerol fatty acid ester may be any known conventional nonionic emulsifier, but is preferably a sucrose fatty acid ester, a glycerol fatty acid ester, an organic acid glycerol fatty acid ester, a sorbitan fatty acid ester, a propylene glycol fatty acid ester, or any of polysorbates, because they can be added to food products, cosmetics, or pharmaceuticals.

In particular, the nonionic emulsifier different from the polyglycerol fatty acid ester is preferably a hydrophilic emulsifier. Herein, an emulsifier having an HLB, which is described in the above, of 10 or more is defined as a hydrophilic emulsifier. Examples of hydrophilic nonionic emulsifiers include sucrose fatty acid esters and polysorbates.

The sucrose fatty acid ester that may be used in the invention is preferably a fatty acid ester whose fatty acid has 12 or more carbon atoms, more preferably 12 to 20 carton atoms.

Preferred examples of sucrose fatty acid ester include sucrose dioleate, sucrose distearate, sucrose dipalmitate, sucrose dimyristate, sucrose dilaurate, sucrose monooleate, sucrose monostearate, sucrose monopalmitate, sucrose monomyristate, and sucrose monolaurate. Among them, more preferred are sucrose monooleate, sucrose monostearate, sucrose monopalmitate, sucrose monomyristate, and sucrose monolaurate.

In the invention, these sucrose fatty acid esters may be used alone or in any combination.

Examples of commercially available sucrose fatty acid esters include RYOTO SUGAR ESTERs S-070, S-170, S-270, S-370, S-370F, S-570, S-770, S-970, S-1170, S-1170F, S-1570, S-1670, P-070, P-170, P-1570, P-1670, M-1695, 0-170, 0-1570, OWA-1570, L-195, L-595, L-1695, LWA-1570, B-370, B-370F, ER-190, ER-290, and POS-135, which are manufactured by Mitsubishi-Kagaku Foods Corporation; and DK ESTERs SS, F160, F140, F110, F90, F70, F50, F-A50, F-20W, F-10, and F-A10E, and COSMELIKEs B-30, S-10, S-50, S-70, S-110, S-160, S-190, SA-10, SA-50, P-10, P-160, M-160, L-10, L-50, L-160, L-150A, L-160A, R-10, R-20, 0-10, and O-150, which are manufactured by DAI-ICHI KOGYO SEIYAKU CO., LTD..

Among the above, preferred are RYOTO SUGAR ESTERs S-1170, S-1170F, S-1570, S-1670, P-1570, P-1670, M-1695, 0-1570, and L-1695, DK ESTER SS, F160, F140, and F110, and COSMELIKEs S-110, S-160, S-190, P-160, M-160, L-160, L-150A, L-160A, and 0-150.

Preferred examples of polysorbates that may be used in an embodiment of the invention include Polysorbate 20 (Polyoxyethylene (20) sorbitan monolaurate), Polysorbate 40 (Polyoxyethylene (20) sorbitan monopalmitate), Polysorbate 60 (Polyoxyethylene (20) sorbitan monostearate), Polysorbate 65 (Polyoxyethylene (20) sorbitan tristearate), Polysorbate 80 (Polyoxyethylene (20) sorbitan monooleate), and Polysorbate 85 (Polyoxyethylene (20) sorbitan trioleate).

In the invention, these polysorbates may be used alone or in any combination.

Examples of commercially available polysorbates include products manufactured by Nikko Chemicals Co., Ltd.: NIKKOL TL-10, NIKKOL TP-10V, NIKKOL TS-10V, NIKKOL TS-10MV, NIKKOL TS-106V, NIKKOL TS-30V, NIKKOL TI-10V, NIKKOL TO-10V, NIKKOL TO-IOMV, NIKKOL TO-106V, and NIKKOL TO-30V.

One kind of the above nonionic emulsifiers different from the polyglycerol fatty acid ester may be used singly or two or more kinds may be used in combination.

If the total content of the nonionic emulsifier(s) different from the polyglycerol fatty acid ester is too low, particles with a small particle diameter may fail to be obtained, and the emulsion may have insufficient stability, and if it is too high, a certain problem may occur, such as vigorous foaming of the emulsion. Therefore, the total nonionic emulsifier content of the emulsion composition is preferably from 0.01% by mass to 8% by mass, more preferably from 0.1% by mass to 5% by mass, even more preferably from 0.5% by mass to 3% by mass.

In the emulsion composition of the invention, the mass ratio (A)/(B) of the amount (A) of the polyglycerol fatty acid ester to the amount (B) of the nonionic emulsifier different from the polyglycerol fatty acid ester needs to be in the range of from 2.5 to 10 and is preferably in the range of from 3 to 9, more preferably in the range of from 4 to 8, because if the amount of the nonionic emulsifier different from the polyglycerol fatty acid ester is too small compared with that of the polyglycerol fatty acid ester, a problem such as precipitation of an oil component may occur during the preparation of the emulsion composition, and if it is too large, a problem such as low stability of the emulsion may occur.

In view of small particle diameter, storage stability of such fine particles, and transparency of the emulsion composition, the emulsion composition of the invention preferably contains from 0.1% by mass to 20% by mass of the polyglycerol fatty acid ester and from 0.01% by mass to 8% by mass of the nonionic emulsifier different from the polyglycerol fatty acid ester, and preferably contains from 0.1% by mass to 5% by mass of the carotenoid, 0.1% by mass to 20% by mass of the polyglycerol fatty acid ester, and 0.01% by mass to 8% by mass of the nonionic emulsifier different from the polyglycerol fatty acid ester.

### [Additional Ingredients]

The emulsion composition of the invention may also contain any of the ingredients described below as an additional ingredient in addition to the oily ingredients, the polyglycerol fatty acid ester, and the nonionic emulsifier different from the polyglycerol fatty acid ester.

### (Polyhydric Alcohol)

The emulsion composition of the invention preferably contains a polyhydric alcohol in view of particle diameter, stability, and antiseptic properties.

Polyhydric alcohols have a moisturizing function, a viscosity controlling function, or the like. Polyhydric alcohols also have the function of reducing the interfacial tension between water and an oil or fat ingredient and facilitating the expansion of the interface so that fine stable particles can be easily formed.

Therefore, the emulsion composition preferably contains a polyhydric alcohol since the emulsion particle diameter can be made smaller and the small particle diameter can be kept stable for a long term.

The addition of a polyhydric alcohol also makes it possible to reduce the water activity of the emulsion composition so that proliferation of microorganisms can be prevented.

Any di- or higher hydric alcohol may be used as the polyhydric alcohol that may be used in the invention, without particular restriction.

Examples of the polyhydric alcohol include glycerol, diglycerol, polyglycerol, 3-methyl-1,3-butanediol, 1,3-butylene glycol, isoprene glycol, polyethylene glycol, 1,2-pentanediol, 1,2-hexanediol, propylene glycol, dipropylene glycol, polypropylene glycol, ethylene glycol, diethylene glycol, pentaerythritol, neopentyl glycol, maltitol, reduced starch syrup, sucrose, lactitol, palatinit, erythritol, sorbitol, mannitol, xylitol, xylose, glucose, lactose, mannose, maltose, galactose, fructose, inositol, pentaerythritol, maltotriose, sorbitol, sorbitan, trehalose, starch sugar, and alcohols produced by reduction of starch sugar. One of these may be used singly or two or more thereof may used in the form of a mixture.

It is preferable to use a polyhydric alcohol having three or more hydroxyl groups per molecule, as the polyhydric alcohol. In this case, the interfacial tension between an aqueous solvent and an oil or fat component can be more efficiently reduced, so that stable and smaller particles can be formed. As a result, high intestinal absorptiveness can be achieved in the case of food applications, and high transdermal absorptiveness can be achieved in the case of cosmetic applications.

Among polyhydric alcohols that satisfy the requirements described above, the use of glycerol is particularly preferred, because the use of glycerol makes it possible to further reduce the particle diameter of the emulsion and to keep the reduced particle diameter stable for a long term.

The total polyhydric alcohol content of the emulsion composition is preferably from 10% by mass to 60% by mass, more preferably from 20% by mass to 55% by mass, even more preferably from 30% by mass to 50% by mass, in view of the viscosity of the emulsion composition as well as the particle diameter, stability, and antiseptic properties mentioned above.

A total polyhydric alcohol content of 10% by mass or more is preferred because at such a content, sufficient storage stability is easily obtained in accordance with the type or content of the oil or fat ingredients. A polyhydric alcohol content of 60% by mass or less is preferred because at such a content, a maximal effect can be obtained, and an increase in the viscosity of the emulsion composition can be easily prevented.

### (Phospholipid)

The emulsion composition of the invention may also contain a phospholipid as an additional ingredient.

As used herein, the term "phospholipids" means a group of esters that are formed from fatty acid(s), alcohol(s), and phosphoric acid(s), and have a phosphate ester moiety and a fatty acid ester moiety. The term "phospholipids" includes glycerophospholipids and sphingophospholipids.

Hereinafter, a detail description is given.

Examples of glycerophospholipids, which are phospholipids that may be used in the invention, include phosphatidic acid, bisphosphatidic acid, lecithin (phosphatidylcholine), phosphatidylethanolamine, phosphatidylmethylethanolamine, phosphatidylserine, phosphatidylinositol, phosphatidylglycerol, and diphosphatidylglycerol (cardiolipin), as well as products containing any of the above and derived from plants such as soy bean, corn, peanut, rape seed, wheat, or the like, products containing any of the above and derived from egg yolk or an animal such as cattle, and various lecithins derived from microorganisms such as Escherichia coli.

Sphingophospholipids, which are phospholipids that may be used in the invention, include sphingomyelin.

In the invention, the glycerophospholipid to be used may be an enzymatically decomposed glycerophospholipid.

For example, lysolecithin, a product of enzymatic decomposition of the lecithin (enzymatically decomposed lecithin), results from the loss of one of the fatty acids (acyl groups), which has been bonded at position 1 or 2 of a glycerophospholipid. When the number of the fatty acid groups is reduced to one, the hydrophilicity of lecithin is improved, and the emulsifying or dispersing ability in water can be improved.

Lysolecithin is obtained by hydrolysis of lecithin in the presence of an acid or alkali catalyst. Alternatively, it may also be obtained by hydrolysis of lecithin with phospholipase A₁ or A₂.

Examples of compound name of such liso compounds typified by lysolecithin include lysophosphatidic acid, lysophosphatidylglycerol, lysophosphatidylinositol, lysophosphatidylethanolamine, lysophosphatidylmethylethanolamine, lysophosphatidylcholine (lysolecithin), and lysophosphatidylserine.

Glycerophospholipids typified by lecithin may be hydrogenated or hydroxylated, which may be used in the invention.

For example, the hydrogenation may be performed by allowing lecithin to react with hydrogen in the presence of a catalyst, whereby the unsaturated bond of the fatty acid moiety is hydrogenated. The hydrogenation improves the stability of lecithin against oxidation.

The hydroxylation may be performed by heating lecithin together with high concentrations of hydrogen peroxide and an organic acid such as acetic acid, tartaric acid, or butyric acid, in which the unsaturated bond of the fatty acid moiety is hydroxylated. The hydroxylation improves the hydrophilicity of lecithin.

In particular, the hydrogenated or hydroxylated lecithin is preferably used in cosmetic applications.

In view of emulsion stability, lecithin or lysolecithin, each of which is a glycerophospholipid, is particularly preferred, and lecithin is more preferred.

The lecithin has a hydrophilic group and a hydrophobic group in the molecule and, therefore, has been widely used as an emulsifier in the fields of food products, pharmaceuticals, and cosmetics.

Lecithin with a purity of 60% by mass or more is industrially used. In the invention, however, lecithin with a purity of 80% by mass or more, which is generally called "high-purity lecithin," is preferred, and lecithin with a purity of 90% by mass or more is more preferred.

The purity of lecithin (% by mass) can be be obtained by subtracting the weights of toluene-insoluble matter and acetone-soluble matter, utilizing the nature of lecithin that it is highly soluble in toluene but insoluble in acetone.

High-purity lecithin is more lipophilic than lysolecithin, and therefore it is considered that compatibility between lecithin and the oily ingredients is high and that emulsion stability is improved.

In the invention, one of the phospholipids may be used singly or two or more thereof may be used in the form of a mixture. When the emulsion composition of the invention contains a phospholipid or phospholipids, the total phospholipid content is preferably from 0.01 times to 0.3 times the total mass of the oily ingredients in the oil phase in view of the transparency and dispersion stability of the emulsion composition.

### [Particle Diameter]

The dispersed particles in the emulsion composition of the invention need to have a volume average particle diameter of 200 nm or less, more preferably have a volume average particle diameter of 120 nm or less, most preferably have a volume average particle diameter of 100 nm or less.

When the particle diameter (volume average particle diameter) of the dispersed particles is 200 nm or less, products produced with the emulsion composition (emulsified product) of the invention, such as food products or cosmetics, are less likely to degrade in transparency or less likely to have reduced internal absorption or transdermal absorption, which is preferred.

The particle diameter of the dispersed particles in the emulsion composition of the invention may be measured with a commercially available particle diameter distribution meter or the like. Known methods for measuring the particle diameter distribution of emulsions include optical microscopy, confocal laser microscopy, electron microscopy, atomic force microscopy, a static light scattering method, a laser diffraction method, a dynamic light scattering method, a centrifugal sedimentation method, electrical pulse measurement, a chromatography method, and a ultrasonic attenuation method. Devices based on respective principles are commercially available.

In the invention, the dynamic light scattering method is preferably used to measure the particle diameter, in view of a range of the particle diameter and ease of measurement. Examples of commercially available devices based on dynamic light scattering include NANOTRAC UPA (trade name, manufactured by NIKKISO CO., LTD.), a dynamic light scattering particle diameter analyzer LB-550 (trade name, manufactured by HORIBA, Ltd.), and a concentrated system particle diameter analyzer FPAR-1000 (trade name, manufactured by OTSUKA ELECTRONICS CO., LTD.).

The particle diameter in the invention is defined as a value measured using a concentrated system particle diameter analyzer FPAR-1000 (trade name, manufactured by OTSUKA ELECTRONICS CO., LTD.), and specifically, the value measured as described below is used.

In the method for measuring the particle diameter, a sample is diluted with pure water so that the concentration of oily ingredients is 1% by mass and, then the diluted sample is measured using a glass cell. The particle diameter can be determined as the median diameter obtained when the refractive index and viscosity of the dispersion medium are set at 1.313 (pure water) and 0.8854 (pure water), respectively.

The particle diameter of the particles in the emulsion composition can be made smaller, depending on not only the above ingredients of the emulsion composition but also other factors such as the temperature for dissolving the oil phase and the aqueous phase, the stirring conditions (shearing force, temperature, and pressure), and the ratio between the oil phase and the aqueous phase in the method described below for producing the emulsion composition.

### <Method for Producing the Emulsion Composition>

For example, the method for producing the emulsion composition of the invention is preferably, but not particularly limited to, a method including the steps of a) dissolving the polyglycerol fatty acid ester and the nonionic emulsifier different from the polyglycerol fatty acid ester in an aqueous medium (such as water) to form an aqueous phase, b) mixing and dissolving the oily ingredients (the high-melting-point carotenoid and so on) to form an oil phase, and c) mixing the aqueous phase and the oil phase under stirring to disperse and emulsify them so that an emulsion composition is obtained.

Particularly, since the high-melting-point carotenoid is included in the invention, the carotenoid should be heat-treated at a temperature equal to or higher than the melting point so that it can be sufficiently dissolved in the oil phase, before mixed with the aqueous phase and subjected to emulsification.

In the production method, the ingredients contained in the oil phase and the aqueous phase are the same as the ingredients of the emulsion composition of the invention described above, and preferred examples and preferred amounts thereof, and more preferred combinations are also the same as those described above.

In the dispersion and emulsification, the ratio (by mass) of the oil phase to the aqueous phase (oil phase/aqueous phase ratio (% by mass)) is preferably, but not particularly limited, from 0.1/99.9 to 50/50, more preferably from 0.5/99.5 to 30/70, even more preferably from 1/99 to 20/80.

When the oil phase/aqueous phase ratio is 0.1/99.9 or more, the active ingredient is not at a low level so that the resulting emulsion composition tends to have no practical problem, which is preferred. When the oil phase/aqueous phase ratio is 50/50 or less, the surfactant concentration is not low so that the emulsion composition does not tend to degrade in emulsion stability, which is preferred.

The dispersion and emulsification may be performed using a one-step emulsification process, but is preferably performed using a two or more-step emulsification process from the viewpoint of obtaining uniform and fine particles.

Specifically, it is particularly preferred to use two or more emulsification devices in combination in such a way that a one-step emulsification process using a general emulsification device utilizing shearing (such as a stirrer, an impeller mixer, a homo mixer, or a continuous-flow shearing apparatus) is performed together with an emulsion process through a high-pressure homogenizer, an ultrasonic disperser, or the like. The use of a high-pressure homogenizer allows the emulsified product to have more uniform droplets of fine particles. Emulsification may also be performed twice or more in order to form droplets with a more uniform particle diameter.

The emulsion composition of the invention may be widely used in beverages and cosmetic products. The cosmetic product is selected from the group consisting of: a skin lotion, an essence, a milky lotion, a cream pack, a mask, a pack, a shampoo cosmetic, a fragrance cosmetic, a liquid body cleanser, a UV-care cosmetic, a deodorant cosmetic and an oral care cosmetic.

If necessary, any appropriate ingredient acceptable for food or cosmetic use may be added to the food or cosmetic product of the invention, which contains the emulsion composition of the invention.

The content of the emulsion composition of the invention in the food product, the cosmetic product, depends on the type or purpose of the product and is not necessarily defined, but the emulsion composition of the invention can be used in the range of from 0.01% by mass to 10% by mass, preferably in the range of from 0.05% by mass to 5% by mass, based on the amount of the product.

If the content is too low, the desired effect may fail to be achieved, and if the content is too high, the emulsion composition added in an excess amount may fail to contribute to the production of the effect.

In particular, when the emulsion composition of the invention is used in an aqueous product such as a beverage (which is a food product), or a skin lotion, an essence, a milky lotion, a cream pack or mask, a pack, a shampoo cosmetic, a fragrance cosmetic, a liquid body cleanser, a UV-care cosmetic, a deodorant cosmetic, or an oral care cosmetic (in the case of a cosmetic product), product having transparent appearance is obtained, and an undesired phenomenon can be prevented, such as precipitation, sedimentation, or a neck ring of an insoluble material under severe conditions such as long-term storage or sterilization.

For example, the food or cosmetic product of the invention may be obtained by a conventional method in which the emulsion composition of the invention and an optional ingredient or ingredients capable of being added are subjected to mixing

### EXAMPLES

Hereinafter, the invention is described specifically with reference to the examples.

### (Examples 1-1 and 1-2 and Comparative Examples 1-1 and 1-2)

High-melting-point carotenoid-containing emulsion compositions 1-A to 1-D were each prepared by the method described below using the composition shown below.

The melting point of the carotenoid was checked as described below according to the melting point-measuring method described above.

Specifically, the ingredients for liquid 1 below were weighed into a vessel according to the composition shown below, and uniformly mixed by stirring, whereby a mixture was obtained. A part of the mixture was used as an oil-based sample in the measurement of the melting point, and the melting point of lycopene in liquid 1 was measured by the method including the steps (1) to (4) described above. As a result, the melting point was found to be 130°C.

**[Liquid 1]**

| | |
|---|---|
| Tomato oleoresin | 3.3% by mass |
| (trade name: LYCOPENE 18, manufactured by KYOWA HAKKO BIO Co., Ltd., containing 18% by mass of lycopene) | |
| Lecithin | 1.0% by mass |
| (trade name: LECION P, manufactured by RIKEN VITAMIN CO., LTD.) | |
| Medium-chain fatty acid glyceride | 10.7% by mass |
| (trade name: COCONARD MT, manufactured by Kao Corporation) | |

**[Liquid 2]**

| | |
|---|---|
| Decaglyceryl oleate-10 | X% by mass |
| (trade name: DECAGLYN 1-O, manufactured by Nikko Chemicals Co., Ltd.) | |
| Sucrose stearate | Y% by mass |
| (trade name: RYOTO SUGAR ESTER S-1670, manufactured by Mitsubishi-Kagaku Foods Corporation) | |
| Glycerol | 45% by mass |
| Purified water | 30% by mass |

**[Table 1]**

| | | Emulsion composition | | | |
|---|---|---|---|---|---|
| | | 1-A (Comparative Example 1-1) | 1-B (Example 1-1) | 1-C (Comparative Example 1-2) | 1-D (Comparative Example 1-2) |
| Decaglyceryl oleate-10 | X (mass %) | 6.7 | 7.3 | 8.0 | 10.0 |
| Sucrose stearate | Y (mass %) | 3.3 | 2.7 | 2.0 | 0.0 |
| X/Y | | 2.03 | 2.70 | 4.00 | - |

The ingredients for liquid 2 above were weighed into a vessel according to the composition shown above (including the composition shown in Table 1), and heated and mixed by stirring in a thermostatic chamber at 70°C. After the well-mixed state was checked, the mixture was kept at 70°C.

The ingredients for liquid 1 above were also weighed into a vessel according to the composition shown above, and heated and mixed by stirring on a hot plate at 150°C for 5 minutes. The well-mixed state was checked.

The liquid 2 obtained as described above was added to the liquid 1, mixed by stirring, and dispersed using an ultrasonic homogenizer.

Subsequently, the resulting coarse dispersion was further subjected to high-pressure emulsification at 200 MPa using an ultra-high pressure emulsifier (trade name: ULTIMIZER, manufactured by SUGINO MACHINE LIMITED).

### (Examples 2-1 to 2-3 and Comparative Examples 2-1 to 2-3)

High-melting-point carotenoid-containing emulsion compositions 2-A to 2-F were each prepared by the same process as in Example 1-1 using the composition shown below (including the composition shown in Table 2).

The melting point of the carotenoid was checked as described below according to the melting point-measuring method described above.

Specifically, the ingredients for liquid 1 below were weighed into a vessel according to the composition shown below, and uniformly mixed by stirring, whereby a mixture was obtained. Apart of the mixture was used as an oil-based sample in the measurement of the melting point, and the melting point of lycopene in liquid 1 was measured by the method including the steps (1) to (4) described above. As a result, the melting point was found to be 130°C.

**[Liquid 1]**

| | |
|---|---|
| Tomato oleoresin | 3.3% by mass |
| (trade name: LYC-O-MATO 15%, manufactured by SUN BRIGHT CO., LTD., containing 15% by mass of lycopene) | |
| Lecithin | 1.0% by mass |
| (trade name: LECION P, manufactured by RIKEN VITAMIN CO., LTD.) | |
| Medium-chain fatty acid glyceride | 10.7% by mass |
| (trade name: COCONARD MT, manufactured by Kao Corporation) | |

**[Liquid 2]**

| | |
|---|---|
| Decaglyceryl oleate-10 | X% by mass |
| (trade name: DECAGLYN 1-O, manufactured by Nikko Chemicals Co., Ltd.) | |
| Sucrose stearate | Y% by mass |
| (trade name: RYOTO SUGAR ESTER S-1670, manufactured by Mitsubishi-Kagaku Foods Corporaton) | |
| Glycerol | 45% by mass |
| Purified water | 30% by mass |

**[Table 2]**

| | | Emulsion composition | | | | | |
|---|---|---|---|---|---|---|---|
| | | 2-A (Comparative Example 2-1) | 2-B (Example 2-1) | 2-C (Example 2-2) | 2-D (Example 2-3) | 2-E (Comparative Example 2-2) | 2-F (Comparative Example 2-3) |
| Decaglyceryl oleate-10 | X (mass %) | 6.7 | 7.3 | 8.0 | 9.0 | 9.2 | 10.0 |
| Sucrose stearate | Y (mass %) | 3.3 | 2.7 | 2.0 | 1.0 | 0.8 | 0.0 |
| X/Y | | 2.03 | 2.70 | 4.00 | 9.00 | 11.50 | - |

### (Comparative Examples 3-1 to 3-5)

Carotenoid-containing emulsion compositions 3-A to 3-E were each prepared by the same process as in Example 1-1 using the composition shown below.

**[Liquid 1]**

| | |
|---|---|
| Tomato oleoresin | 3.3% by mass |
| (trade name: LYC-O-MATO 15%, manufactured by SUN BRIGHT CO., LTD., containing 15% by mass of lycopene) | |
| Lecithin | 1.0% by mass |
| (trade name: LECION P, manufactured by RIKEN VITAMIN CO., LTD.) | |
| Medium-chain fatty acid glyceride | 10.7% by mass |
| (trade name: COCONARD MT, manufactured by Kao Corporation) | |

**[Liquid 2]**

| | |
|---|---|
| Sucrose fatty acid ester | 10% by mass |
| (trade name: RYOTO SUGAR ESTER shown in Table 3, manufactured by Mitsubishi-Kagaku Foods Corporation.) | |
| Glycerol | 45% by mass |
| Purified water | 30% by mass |

**[Table 3]**

| Emulsion composition | Sucrose fatty acid ester |
|---|---|
| 3-A (Comparative Example 3-1) | Sucrose stearate (RYOTO SUGAR ESTER S-1670) |
| 3-B (Comparative Example 3-2) | Sucrose oleate (RYOTO SUGAR ESTER O-1570) |
| 3-C (Comparative Example 3-3) | Sucrose palmitate (RYOTO SUGAR ESTER P-1670) |
| 3-D (Comparative Example 3-4) | Sucrose myristate (RYOTO SUGAR ESTER M-1695) |
| 3-E (Comparative Example 3-5) | Sucrose laurate (RYOTO SUGAR ESTER L-1695) |

### (Examples 4-1 to 4-3)

High-melting-point carotenoid-containing emulsion compositions 4-A to 4-C were each prepared by the same process as in Example 1-1 using the composition shown below.

The melting point of the carotenoid used in Example 4-B was checked as described below according to the melting point-measuring method described above.

Specifically, the ingredients for liquid 1 below were weighed into a vessel according to the composition shown below, and uniformly mixed by stirring, whereby a mixture was obtained. A part of the mixture was used as an oil-based sample in the measurement of the melting point, and the melting point of β-carotene in liquid 1 was measured by the method including the steps (1) to (4) described above. As a result, the melting point was found to be 140°C.

The melting point of the carotenoid used in Example 4-C was checked as described below according to the melting point-measuring method described above.

Specifically, the ingredients for liquid 1 below were weighed into a vessel according to the composition shown below, and uniformly mixed by stirring, whereby a mixture was obtained. A part of the mixture was used as an oil-based sample in the measurement of the melting point, and the melting point of the carotenoid in liquid 1 was measured by the method including the steps (1) to (4) described above. As a result, the melting point was found to be 100°C.

### <4-A>

**[Liquid 1]**

| | |
|---|---|
| Tomato oleoresin (trade name: LYC-O-MATO 15%, manufactured by SUN BRIGHT CO., LTD., containing 15% by mass of lycopene) | 3.4% bymass |
| Mixed tocopherol (trade name: RIKEN E OIL 800, manufactured by RIKEN VITAMIN CO., LTD.) | 0.6% bymass |
| Medium-chain fatty acid glyceride (trade name: COCONARD MT, manufactured by Kao Corporation) | 11% bymass |

**[Liquid 2]**

| | |
|---|---|
| Decaglyceryl oleate-10 (trade name: DECAGLYN 1-O, manufactured by Nikko Chemicals Co., Ltd.) | 8% by mass |
| Sucrose stearate (trade name: RYOTO SUGAR ESTER S-1670, manufactured by Mitsubishi-Kagaku Foods Corporation.) | 2% bymass |
| Glycerol | 45% by mass |
| Purified water | 30% by mass |

### <4-B>

**[Liquid 1]**

| | |
|---|---|
| Dunaliella extract (trade name: BIO CAROTENE 30MCT, manufactured by KYOWA HAKKO BIO Co., Ltd., | 1.7% by mass |
| containing of β-carotene) Mixed tocopherol 0.6% by mass | 30% by mass |
| (trade name: RIKEN E OIL 800, manufactured by RIKEN VITAMIN CO., LTD.) Medium-chain fatty acid glyceride | 12.7% by mass |
| (trade name: COCONARD MT, manufactured by Kao Corporation) | |

**[Liquid 2]**

| | |
|---|---|
| Decaglyceryl oleate-10 (trade name: DECAGLYN 1-O, manufactured by Nikko Chemicals Co., Ltd.) | 8% by mass |
| Sucrose stearate (trade name: RYOTO SUGAR ESTER S-1670, manufactured by Mitsubishi-Kagaku Foods Corporation.) | 2% by mass |
| Glycerol | 45% by mass |
| Purified water | 30% by mass |

### <4-C>

**[Liquid 1]**

| | |
|---|---|
| Multi carotenoid suspension (trade name: NATURAL MULTI CAROTENOID 20, manufactured by KYOWA HAKKO BIO Co., Ltd., 20% by mass in total carotenoid content) | 1.7% bymass |
| Mixed tocopherol (trade name: RIKEN E OIL 800, manufactured by RIKEN VITAMIN CO., LTD.) | 1.0% by mass |
| Medium-chain fatty acid glyceride (trade name: COCONARD MT, manufactured by Kao Corporation) | 12.3% bymass |

**[Liquid 2]**

| | |
|---|---|
| Decaglyceryl oleate-10 (trade name: DECAGLYN 1-O, manufactured by Nikko Chemicals Co., Ltd.) | 8% by mass |
| Sucrose stearate (trade name: RYOTO SUGAR ESTER S-1670, manufactured by Mitsubishi-Kagaku Foods Corporation.) | 2% by mass |
| Glycerol | 45% by mass |
| Purified water | 30% by mass |

### (Comparative Example 5)

A carotenoid-containing emulsion composition 5-A was prepared by the method described below.

Specifically, the emulsion composition was prepared by the same process with the same composition as in Example 1-1, except that the ingredients for liquid 1 were heated and mixed by stirring on a hot plate at 100°C for 5 minutes instead of heating and mixing by stirring on a hot plate at 150°C for 5 minutes.

### <Evaluation>

### (Particle Diameter and Storage Stability of Particles)

The resulting emulsion compositions (1-A to 1-D, 2-A to 2-F, 3-A to 3-E, 4-A to 4-C, and 5-A) were each divided into three samples. The samples were each placed in a 5 ml glass vial, and one is stored at 4°C for 2 weeks, and another was stored at 4°C for a month. Purified water was added to each of the samples before the storage, after the 2 week storage, and after the one month storage to form a 1% dilution. The volume average particle diameter (median particle diameter) of the particles in each dilution was measured with a dynamic light scattering meter. The measurement results are summarized in Table 4.

Based on the measurement results, each emulsion composition was scored according to the criteria shown below, and the results are also shown in Table 4 below. The particle diameter values in the table are in units of nm.

### <Evaluation Criteria>

1: The particle diameter after the 2 week storage is more than 200 nm.
2: The particle diameters before the storage and after the 2 week storage are 200 nm or less, and the particle diameter after the one month storage is more than 200 nm.
3: The particle diameters before the storage and after the 2 week storage are 200 nm or less, and the particle diameter after the one month storage is 200 nm or less.
4: The particle diameter before the storage is 100 nm or less, the particle diameter after the 2 week storage is 100 nm or less, and the particle diameter after the one month storage is more than 100 nm and 200 nm or less.
5: The particle diameter before the storage is 100 nm or less, the particle diameter after the 2 week storage is 100 nm or less, and the particle diameter after the one month storage is 100 nm or less.

**[Table 4]**

| | Emulsion composition | Particle diameter | | | Score |
|---|---|---|---|---|---|
| | | Before storage | After 2 week storage | After 1 month storage | |
| Compartive Example 1-1 | 1-A | 65.1 | 118 | 135 | 3 |
| Example 1-1 | 1-B | 46.9 | 56.5 | 56.9 | 5 |
| Example 1-2 | 1-C | 50.3 | 54.6 | 51.6 | 5 |
| Compartive Example 1-2 | 1-D | >1000 | >1000 | >1000 | 1 |
| Compartive Example 2-1 | 2-A | 77.4 | 111 | 113 | 3 |
| Example 2-1 | 2-B | 73.7 | 72.4 | 79.9 | 5 |
| Example 2-2 | 2-C | 73.7 | 68.6 | 72.8 | 5 |
| Example 2-3 | 2-D | 67.1 | 72.0 | 69.4 | 5 |
| Compartive Example 2-2 | 2-E | >1000 | >1000 | >1000 | 1 |
| Compartive Example 2-3 | 2-F | >1000 | >1000 | >1000 | 1 |
| Compartive Example 3-1 | 3-A | 116 | 355 | >1000 | 1 |
| Compartive Example 3-2 | 3-B | 587 | >1000 | >1000 | 1 |
| Compartive Example 3-3 | 3-C | 54.2 | 299 | >1000 | 1 |
| Compartive Example 3-4 | 3-D | 37.7 | 740 | >1000 | 1 |
| Compartive Example 3-5 | 3-E | 43.9 | 43.8 | >1000 | 2 |
| Example 4-1 | 4-A | 74.2 | 73.1 | 82.4 | 5 |
| Example 4-2 | 4-B | 92.9 | 80.1 | 80.0 | 5 |
| Example 4-3 | 4-C | 95.4 | 99.9 | 105.6 | 4 |
| Compartive Example 5 | 5-A | >1000 | >1000 | >1000 | 1 |

### (Evaluation of Transparency)

After the one month storage, the 1% dilution of each emulsion composition, which was prepared for the evaluation of the storage stability, was placed in a vessel with a height of 5 cm or more in such a manner that the liquid level was 4 cm above the bottom of the vessel. The sample liquid was visually observed from vertically above the liquid surface toward the bottom of the vessel. In this manner, whether the bottom of the vessel was visible was evaluated as an evaluation for transparency according to the criteria shown below. The results are shown in Table 5 below.

### <Evaluation Criteria>

**[Table 5]**

| | Emulsion composition | Visual evaluation of transparency |
|---|---|---|
| Compartive Example 1-1 | 1-A | - |
| Example 1-1 | 1-B | ++ |
| Example 1-2 | 1-C | ++ |
| Compartive Example 1-2 | 1-D | - |
| Compartive Example 2-1 | 2-A | + |
| Example 2-1 | 2-B | ++ |
| Example 2-2 | 2-C | ++ |
| Example 2-3 | 2-D | ++ |
| Compartive Example 2-2 | 2-E | - |
| Compartive Example 2-3 | 2-F | - |
| Compartive Example 3-1 | 3-A | - |
| Compartive Example 3-2 | 3-B | - |
| Compartive Example 3-3 | 3-C | - |
| Compartive Example 3-4 | 3-D | - |
| Compartive Example 3-5 | 3-E | - |
| Example 4-1 | 4-A | ++ |
| Example 4-2 | 4-B | ++ |
| Example 4-3 | 4-C | + |
| Compartive Example 5 | 5-A | - |

| | | |
|---|---|---|
| ++: The bottom is clearly visible. +: The bottom is vaguely visible. -: The bottom is invisible. | | |

From the above, it is shown that in all of the emulsion compositions of the examples, each of which contains a high-melting-point carotenoid in the oil phase and a polyglycerol fatty acid ester and a nonionic emulsifier (sucrose fatty acid ester) different from the polyglycerol fatty acid ester in a specific content ratio ((A)/(B) = from 2.5 to 10) in the aqueous phase, the particle diameter is small (100 µm or less) before the storage, and even after the 2 week storage and after the one month storage, and it is possible to suppress increase in the particle diameter and successfully keep the particle diameter small.

As shown in Table 5, it is shown that as a result of visual observation, the emulsion compositions of the examples each can be found to have very high transparency when diluted to 1% with purified water. There is a correlation between the transparency and the particle diameter of the particles in the emulsion composition, and high transparency can be observed when the particle diameter is preferably 120 nm or less, most preferably 100 nm or less.

## Claims

1. An emulsion composition obtained by mixing an oil phase comprising a carotenoid having a melting point of 100 °C or more as detected when it is comprised in an oil; and an aqueous phase comprising a polyglycerol fatty acid ester and a nonionic emulsifier that is different from the polyglycerol fatty acid ester, wherein:
a weight ratio (A)/(B) of an amount (A) of polyglycerol fatty acid ester contained in the emulsion composition to an amount (B) of nonionic emulsifier other than polyglycerol fatty acid ester, that is contained in the emulsion composition is in a range of from 2.5 to 10; and
a particle diameter of dispersed particles that comprise the carotenoid is 200 nm or less.

2. The emulsion composition of claim 1, which comprises from 0.1% by mass to 20% by mass of the polyglycerol fatty acid ester and from 0.01% by mass to 8% by mass of the nonionic emulsifier that is different from the polyglycerol fatty acid ester.

3. The emulsion composition of claim 1, which comprises from 0.1% by mass to 5% by mass of the carotenoid, from 0.1% by mass to 20% by mass of the polyglycerol fatty acid ester, and from 0.01% by mass to 8% by mass of the nonionic emulsifier that is different from the polyglycerol fatty acid ester.

4. The emulsion composition of any one of claims 1 to 3, wherein the carotenoid is lycopene.

5. The emulsion composition of any one of claims 1 to 4, wherein the nonionic emulsifier that is different from the polyglycerol fatty acid ester is at least one selected from the group consisting of sucrose fatty acid esters, glycerol fatty acid esters, organic acid glycerol fatty acid esters, sorbitan fatty acid esters, propylene glycol fatty acid esters and polysorbates.

6. The emulsion composition of any one of claims 1 to 5, further comprising a phospho lipid.

7. The emulsion composition of any one of claims 1 to 6, further comprising an antioxidant.

8. The emulsion composition of any one of claims 1 to 7, further comprising a polyhydric alcohol.

9. The emulsion composition of any one of claims 1 to 8, wherein the particle diameter is 100 nm or less.

10. Use of the emulsion composition of any one of claims 1 to 9 for the production of a beverage or a cosmetic product which is an aqueous product selected from the group consisting of: a skin lotion, an essence, a milky lotion, a cream pack, a mask, a pack, a shampoo cosmetic, a fragrance cosmetic, a liquid body cleanser, a UV-care cosmetic, a deodorant cosmetic and an oral care cosmetic.

## Patentansprüche

1. Emulsionszusammensetzung erhalten durch Mischen einer ein Carotinoid mit einem Schmelzpunkt von 100 °C oder mehr ermittelt, wenn es in einem Öl enthalten ist, enthaltenden Ölphase; und einer einen Polyglycerinfettsäureester und einen nichtionischen Emulgator, der sich von dem Polyglycerinfettsäureester unterscheidet, enthaltende Wasserphase, wobei:
ein Gewichtsverhältnis (A)/(B) einer Menge (A) des in der Emulsionszusammensetzung enthaltenen Polyglycerinfettsäureesters zu einer Menge (B) des in der Emulsionszusammensetzung enthaltenen nichtionischen Emulgators, der sich von dem Polyglycerinfettsäureester unterscheidet, in einem Bereich von 2,5 bis 10 liegt; und
ein Partikeldurchmesser von dispergierten Partikeln, die das Carotinoid enthalten, 200 nm oder weniger beträgt.

2. Emulsionszusammensetzung nach Anspruch 1, die 0,1% Masse% bis 20 Masse% des Polyglycerinfettsäureesters und 0,01 Masse% bis 8 Masse% des nichtionischen Emulgators, der sich von dem Polyglycerinfettsäureester unterscheidet, enthält.

3. Emulsionszusammensetzung nach Anspruch 1, die 0,1 Masse% bis 5 Masse% des Carotinoids, 0,1 Masse% bis 20 Masse% des Polyglycerinfettsäureesters und 0,01 Masse% bis 8 Masse% des nichtionischen Emulgators, der sich von dem Polyglycerinfettsäureester unterscheidet, enthält.

4. Emulsionszusammensetzung nach einem der Ansprüche 1 bis 3, wobei das Carotinoid Lycopen ist.

5. Emulsionszusammensetzung nach einem der Ansprüche 1 bis 4, wobei der nichtionische Emulgator, der sich von dem Polyglycerinfettsäureester unterscheidet, mindestens einer, ausgewählt aus der Gruppe bestehend aus Saccharosefettsäureestern, Glycerinfettsäureestern, Glycerinfettsäureestern organischer Säuren, Sorbitanfettsäureestern, Propylenglycolfettsäureestern und Polysorbaten ist.

6. Emulsionszusammensetzung nach einem der Ansprüche 1 bis 5, ferner enthaltend ein Phospholipid.

7. Emulsionszusammensetzung nach einem der Ansprüche 1 bis 6, ferner enthaltend ein Antioxidans.

8. Emulsionszusammensetzung nach einem der Ansprüche 1 bis 7, ferner enthaltend einen mehrwertigen Alkohol.

9. Emulsionszusammensetzung nach einem der Ansprüche 1 bis 8, wobei der Partikeldurchmesser 100 nm oder weniger beträgt.

10. Verwendung der Emulsionszusammensetzung nach einem der Ansprüche 1 bis 9 zur Herstellung eines Getränks oder eines kosmetischen Produkts, das ein wässriges Produkt, ausgewählt aus der Gruppe bestehend aus: einer Hautlotion, einer Essenz, einer milchigen Lotion, einer Crèmepackung, einer Maske, einer Packung, einem kosmetischen Shampoo, einem kosmetischen Duftstoff, einem flüssigen Körperreinigungsmittel, einer UV-Pflegekosmetik, einem kosmetischen Deodorant und einer kosmetischen Oralpflege ist.

## Revendications

1. Composition d'émulsion obtenue en mélangeant une phase huileuse comprenant un caroténoïde présentant un point de fusion de 100 °C ou plus comme détecté lorsqu'il est compris dans une huile, et une phase aqueuse comprenant un ester polyglycérolique d'acides gras et un émulsifiant non ionique, différent de l'ester polyglycérolique d'acides gras, dans lequel :
un rapport en poids (A) / (B) entre une quantité (A) d'ester polyglycérolique d'acides gras contenu dans la composition d'émulsion et une quantité (B) d'émulsifiant non ionique autre que l'ester polyglycérolique d'acides gras, lequel est contenu dans la composition d'émulsion, est compris dans une plage allant de 2,5 à 10, et
un diamètre de particules de particules dispersées, lesquelles comprennent le caroténoïde, est inférieur ou égal à 200 nm.

2. Composition d'émulsion selon la revendication 1, laquelle comprend de 0,1 % en masse à 20 % en masse de l'ester polyglycérolique d'acides gras, et de 0,01 % en masse à 8 % en masse de l'émulsifiant non ionique, lequel est différent de l'ester polyglycérolique d'acides gras.

3. Composition d'émulsion selon la revendication 1, laquelle comprend de 0,1 % en masse à 5 % en masse du caroténoïde, de 0,1 % en masse à 20 % en masse de l'ester polyglycérolique d'acides gras, et de 0,01 % en masse à 8 % en masse de l'émulsifiant non ionique, lequel est différent de l'ester polyglycérolique d'acides gras.

4. Composition d'émulsion selon l'une quelconque des revendications 1 à 3, dans laquelle le caroténoïde est du lycopène.

5. Composition d'émulsion selon l'une quelconque des revendications 1 à 4, dans laquelle l'émulsifiant non ionique, lequel est différent de l'ester polyglycérolique d'acides gras, est au moins un élément sélectionné parmi le groupe consistant en des esters d'acides gras de sucrose, des esters glycéroliques d'acides gras, des esters glycéroliques d'acides gras organiques, des esters d'acides gras de sorbitan, des esters d'acides gras de propylène glycol, et des polysorbates.

6. Composition d'émulsion selon l'une quelconque des revendications 1 à 5, comprenant en outre un phospholipide.

7. Composition d'émulsion selon l'une quelconque des revendications 1 à 6, comprenant en outre un antioxydant.

8. Composition d'émulsion selon l'une quelconque des revendications 1 à 7, comprenant en outre un polyol.

9. Composition d'émulsion selon l'une quelconque des revendications 1 à 8, dans laquelle le diamètre de particules est inférieur ou égal à 100 nm.

10. Utilisation de la composition d'émulsion selon l'une quelconque des revendications 1 à 9 pour la production d'une boisson ou d'un produit cosmétique, qui est un produit aqueux sélectionné parmi le groupe consistant en : une lotion pour la peau, une essence, une lotion de type lait, une compresse de crème, un masque, une compresse, un cosmétique de type shampoing, un cosmétique de type parfum, un nettoyant liquide pour le corps, un cosmétique de soin anti-UV, un cosmétique de type déodorant, et un cosmétique de type soin oral.
